**Europäisches Patentamt**

**European Patent Office**

**Office européen des brevets**

(19)

(11) Numéro de publication : **0 304 374 B1**

(12) **FASCICULE DE BREVET EUROPEEN**

(45) Date de publication du fascicule du brevet :
**08.01.92 Bulletin 92/02**

(51) Int. Cl.⁵ : **A61K 47/42, A61K 31/415**

(21) Numéro de dépôt : **88402117.1**

(22) Date de dépôt : **17.08.88**

(54) **Préparations médicamenteuses à base de collagène et de dérivés imidazolés.**

(30) Priorité : **17.08.87 FR 8711649**

(43) Date de publication de la demande :
**22.02.89 Bulletin 89/08**

(45) Mention de la délivrance du brevet :
**08.01.92 Bulletin 92/02**

(84) Etats contractants désignés :
**AT BE CH DE ES FR GB GR IT LI LU NL SE**

(56) Documents cités :
**EP-A- 0 138 216**
**EP-A- 0 224 453**
**EP-A- 0 233 429**

(73) Titulaire : **SEPTODONT**
**58, rue du Pont de Créteil**
**F-94107 Saint Maur des Fosses (FR)**

(72) Inventeur : **Chodkiewicz, Marc Marie Xavier**
**35, rue Beaubourg**
**F-75003 Paris (FR)**
Inventeur : **Leblanc, Dominique Rolande**
**Fernande Marie**
**30, rue Léon Bocquet**
**F-94100 Saint-Maur (FR)**
Inventeur : **Pellion, Brigitte Marie Louise**
**47, avenue de la Division Leclerc**
**F-92320 Chatillon (FR)**

(74) Mandataire : **Martin, Jean-Jacques et al**
**Cabinet REGIMBEAU 26, Avenue Kléber**
**F-75116 Paris (FR)**

**EP 0 304 374 B1**

## Description

La présente invention concerne un procédé de fabrication de préparations médicamenteuses solides associant du collagène et un agent anti-infectieux imidazolé, ainsi que leur application au traitement de diverses affections du parodonte, en particulier des poches parodontales.

Dans l'état de la technique, il a déjà été suggéré d'associer du collagène à divers principes actifs médicamenteux, en particulier à certains agents antiseptiques et/ou anti-inflammatoires, notamment pour le traitement d'affections périodontales.

Il a été proposé par le document EP-A-233429 un procédé de préparation d'une composition pharmaceutique contenant une association de collagène et d'un principe actif antiseptique et/ou anti-inflammatoire, faisant intervenir les étapes suivantes :

a) solubilisation du collagène dans de l'acide acétique,
b) solubilisation du principe actif dans de l'eau distillée,
c) mélange des deux solutions et élimination du solvant.

Cependant, jusqu'à ce jour, il s'est avéré impossible, à l'échelon industriel, de fabriquer dans des conditions techniques et/ou économiques satisfaisantes un tel type de produit qui présente une répartition homogène de principe actif au sein de la masse de collagène et qui puisse être utilisé avec succès, en particulier pour le traitement d'affections parodontales.

La présente invention a précisément eu pour but de mettre au point un procédé pour la fabrication de préparations médicamenteuses solides, qui puisse être mis en oeuvre à l'échelle industrielle et qui conduise à des préparations solides présentant toutes les qualités nécessaires à leur application pharmaceutique. Il est en particulier indispensable d'obtenir de façon constante une répartition homogène de l'agent imidazolé dans la masse de collagène qui ne doit, de son côté, subir aucune dénaturation au cours des diverses étapes de la fabrication.

Conformément à la présente invention, le procédé de fabrication selon l'invention est caractérisé en ce que l'on réalise les opérations successives suivantes :

* on dissout une quantité prédéterminée d'au moins un dérivé imidazolé dans de l'eau déminéralisée,
* dans la solution ainsi obtenue on disperse une quantité prédéterminée de collagène fibreux non-dénaturé, et
* on lyophilise la dispersion ainsi obtenue.

Selon une autre caractéristique de la présente invention, on utilise, pour une partie en masse de collagène fibreux non-dénaturé 0,005 à 0,25 partie en masse de dérivé imidazolé, et 45 à 200 parties en masse d'eau déminéralisée.

D'autres caractéristiques et avantages de la présente invention apparaîtront à la lecture de la description détaillée faite ci-après notamment en s'appuyant sur un exemple de mise en oeuvre particulier, donné à simple titre d'illustration, du procédé de l'invention.

La première étape du procédé de fabrication selon l'invention consiste à dissoudre une quantité prédéterminée d'au moins un agent imidazolé dans de l'eau déminéralisée. En raison de l'application préférentielle des produits selon l'invention, c'est-à-dire le traitement des affections parodontales, l'agent anti-infectieux est choisi de préférence dans la famille des nitro-5-imidazoles, en particulier parmi le métronidazole, l'ornidazole et le tinidazole.

En effet, dans les poches parodontales en évolution, on trouve une flore microbienne variée associant des germes aérobies et anaérobies, Gram-positifs et Gram-négatifs et souvent aussi divers protozoaires, tels qu'Entamoeba gingivalis et Trichomonas tenax. La plupart des spécialistes du traitement des affections du parodonte s'accordent sur l'importance déterminante de la flore anaérobie, et notamment des germes anaérobies mobiles, dans l'étiologie et dans l'évolution clinique des poches parodontales. Un ensemble de données cliniques et expérimentales milite en faveur du rôle d'Entamoeba gingivalis et de Trichomonas tenax dans l'apparition des épisodes aigus qui émaillent l'évolution généralement chronique des poches parodontales.

A côté du traitement mécanique (surfaçage radiculaire) et chirurgical (débridement des lésions profondes), le traitement des poches parodontales fait appel à l'utilisation d'agents antibactériens convenablement choisis. De façon idéale, ceux-ci doivent être actifs, aux concentrations libérées in situ, sur les bactéries anaérobies pathogènes et sur les protozoaires responsables de l'affection traitée.

Parmi les agents anti-infectieux connus et déjà utilisés en thérapeutique, certains composés de la classe des imidazolés possèdent le spectre anti-microbien souhaité ; ce sont notamment le métronidazole, l'ornidazole et le tinidazole largement utilisés dans le traitement des affections à Trichomonas (trichomonases urogénitales), à Lamblia (lambliases), à Entamoeba (amibiases) et dans le traitement local et général des infections causées chez l'homme et chez les animaux par des germes anaérobies (gangrènes, septicémies à germes anaérobies).

2

Pour le traitement local ou général des poches parodontales les imidazolés sont à préférer aux agents antibactériens à large spectre qui sont relativement peu actifs sur les germes anaérobies et inactifs sur les protozoaires pathogènes souvent associés à la flore bactérienne. Il en est ainsi de la chlorhexidine et de ses sels utilisés par voie locale ou des tétracyclines utilisées par voie générale ou locale qui, en outre, comportent l'inconvénient de colorer les dents.

En raison des propriétés d'hydrosolubilité de tous ces dérivés imidazolés, leur dissolution dans l'eau déminéralisée ne soulève aucune difficulté. Dans la pratique, il s'est avéré que l'on obtenait une parfaite dissolution par simple agitation pendant une durée de quelques minutes. La quantité d'eau utilisée pour cette première étape de dissolution n'est donc pas critique. De façon avantageuse, on utilise de l'eau déminéralisée qui a en outre été filtrée sur un filtre de porosité 0,22 μm.

Il s'est avéré de façon tout à fait surprenante que par introduction directe de fibres de collagène dans cette solution aqueuse de dérivé imidazolé, il était possible d'obtenir une répartition homogène du dérivé imidazolé au sein de la masse de collagène fibreux. La dispersion obtenue se prête directement à une lyophilisation pour conduire, de façon simple mais parfaitement fiable, aux préparations médicamenteuses recherchées.

Pour obtenir les effets biologiques attendus de l'association objet de la présente invention, le collagène utilisé doit répondre à des spécifications précises qui portent, entre autres, sur deux points essentiels :

— la non-dénaturation du collagène, c'est dire le maintien de la structure moléculaire de la protéine native, et

— le maintien du caractère fibreux du collagène.

On sait en effet que la molécule de collagène natif comporte une partie centrale caractérisée par trois chaînes polypeptidiques (chaînes α) de 100.000 daltons chacune, assemblées en forme d'hélices gauches dont les axes s'enroulent en superhélices droites autour d'un axe commun.

La dénaturation, par la chaleur par exemple, fait disparaître cette structure hélicoïdale caractéristique.

La non-dénaturation du collagène est appréciée par deux techniques faisant appel à des propriétés physiques différentes de la molécule.

1/ La diffraction de rayons X qui met en évidence le maintien de la structure hélicoïdale.

2/ La calorimétrie différentielle programmée (C D P) qui mesure les quantités de chaleur absorbées lorsque les fibres de collagène, soumises à une augmentation linéraire de température, perdent leur structure hélicoïdale.

Il convient en outre de préciser que par collagène fibreux, on entend désigner un collagène comportant une faible proportion acidosoluble et doté d'une bonne dispersibilité dans l'eau.

Dans le cadre de la présente invention on fera donc appel à un collagène insoluble dans l'eau, hydrodispersible et ne présentant qu'une faible fraction acido-soluble, de préférence de l'ordre de 10 à 15% en masse.

Dans un mode de réalisation préférentiel mais non-limitatif de l'invention, le collagène est extrait de la peau de jeunes bovins suivant la technique générale décrite dans le brevet français n° 1568829. Il est obtenu sous formes de fibres qu'on soumet, avant utilisation suivant la présente invention, à un ensemble de contrôles. Ces contrôles comportent notamment :

— le contrôle de la dispersibilité des fibres dans l'eau et l'acide chlorhydrique dilué : dans cet essai, le collagène doit se montrer très peu soluble et facilement dispersible,

— la calorimétrie différentielle programmée, et la diffraction de rayons X, confirmant le maintien de la structure hélicoïdale.

Ainsi s'assure-t-on du caractère pratiquement non-dénaturé, et essentiellement non-acidosoluble, du collagène utilisé dans le cadre de la présente invention. Ces propriétés du collagène sont nécessaires pour obtenir une bonne hydrodispersibilité lors de la mise en oeuvre de la seconde étape du procédé selon l'invention.

On obtient une dispersion des fibres de collagène dans la solution du dérivé imidazolé, qui peut ultérieurement être soumise dans de bonnes conditions à l'opération de lyophilisation, en utilisant de préférence, pour une partie en masse de collagène fibreux non-dénaturé 0,005 à 0,25 partie en masse de dérivé imidazolé, et 45 à 200 parties en masse d'eau déminéralisée.

De façon avantageuse, la dispersion des fibres de collagène dans la solution de dérivé imidazolé préalablement préparée, est effectuée dans une cuve de mélange, par exemple en acier inoxydable, équipée d'une turbine de défloculation. Cette dispersion est effectuée pendant une durée supérieure ou égale à 15 minutes à une température restant inférieure à 30°C. Habituellement cette dispersion sera effectuée à la température ambiante.

La mise en forme des préparations selon l'invention est obtenue simultanément à l'opération de lyophilisation. De façon avantageuse on répartit, à poids constant, la dispersion de fibres de collagène dans la solution de dérivé imidazolé sur des plateaux, sur lesquels on applique ensuite des grilles de séparation. Les plateaux ainsi préparés sont ensuite introduits dans un lyophilisateur. Dans la pratique, les paramètres suivants de lyophilisation ont conduit à des résultats entièrement satisfaisants :

— température de congélation : –30 ± 5°C
— durée de cycle de lyophilisation : ≧ à 18 heures
— température de désorption : 28 ± 5°C.

Les préparations pharmaceutiques solides ainsi préparées selon le procédé de l'invention, peuvent être obtenues sous diverses formes, en particulier sous forme de compresses. Ces préparations sont de préférence stérilisées par un rayonnement ionisant, directement dans leur conditionnement ultime.

A titre d'exemple non-limitatif, on indiquera ci-après un exemple de préparation d'une association collagène + métronidazole qui se présentera, en fin de production, sous forme de compresses, sèches et stériles, pour application locale.

## EXEMPLE 1

Une fabrication d'essai de l'ordre de 50 g (formule ci-dessous) de l'association collagène-métronidazole a été effectuée en conditions industrielles de façon à obtenir environ 500 compresses.

Métronidazole ....................................................... 2,5 g

Collagène exprimé en fibres sèches .......... 47,5 g

Une solution de métronidazole dans l'eau est préparée par simple mélange pendant 5 minutes de 2,5 g de métronidazole dans 8 litres d'eau déminéralisée et filtrée, 47,5 g de fibres de collagène (exprimé en collagène desséché) sont alors dispersées dans la solution de métronidazole pendant 1 heure. Cette dispersion est répartie sur plateaux inox sur lesquels sont ensuite adaptées des grilles inox dont l'ouverture de maille déterminera la taille des compresses.

Le plateau ainsi préparé est placé dans un lyophilisateur. Les paramètres de lyophilisation sont les suivants :

— température de congélation : –30°C
— durée de cycle de lyophilisation : 24 heures
— température de désorption : 28 ± 5°C.

Au terme de la lyophilisation, on retire la grille, on trie les compresses obtenues, on les conditionne, puis on les stérilise par irradiation gamma à 25000 m$^2 \cdot$ s$^{-2}$ (25 KGy).

La technique décrite dans cet exemple est directement transposable à la production industrielle. Sa mise au point a comporté le contrôle de plusieurs paramètres essentiels dont :

— l'homogénéité de la répartition du métronidazole, au sein d'une même compresse, d'une compresse à l'autre au sein d'un même lot, ou de lots différents ;
— la non-dénaturation du collagène par le rayonnement ionisant utilisé (contrôle effectué par C D P) ;
— la non-dégradation du métronidazole par le rayonnement ionisant utilisé (contrôle effectué par HPLC).

Libération in vitro de l'agent anti-infectieux à partir d'une préparation médicamenteuse fabriquée conformément à l'exemple précédent

Les résultats attendus du traitement par application locale d'une association médicamenteuse réalisée suivant l'invention supposent la libération in situ de l'agent (ou des agents) anti-infectieux associé(s) au collagène. Il est capital de s'assurer par des essais in vitro, avant l'utilisation du produit en thérapeutique humaine, que ces principes actifs anti-infectieux sont effectivement libérés.

Protocole n° 1 :

On utilise comme matériel d'essai des échantillons de compresses préparées suivant la technique décrite ci-dessus, constituées de collagène en fibres (95%) et de métronidazole (5%), les pourcentages étant rapportés au poids sec.

On place ces compresses au contact de la surface d'un milieu de récepteur : 200 ml de solution tampon phosphate isotonique de pH 7,35 maintenue à 37°C au bain marie, et homogénéisé par agitation permanente. Des prélèvements sont effectués régulièrement toutes les 10 minutes et le dosage spectrophotométrique du métronidazole est réalisé par mesure de l'absorption à 320 nm.

Les résultats obtenus, consignés dans le tableau ci-dessous, démontrent que la libération du métronidazole à partir de compresses est complète et rapide.

## Libération du métronidazole à partir de compresses de collagène + métronidazole

| TEMPS DE LIBERATION | Pourcentage de métronidazole libéré (quantités cumulées) | | |
|---|---|---|---|
| | Moyenne de 6 essais | Ecart type | coefficient de variation |
| 10 min. | 61,2 % | 12,0 | 19,6 % |
| 20 min. | 80,5 % | 13,5 | 15,9 % |
| 30 min. | 92,0 % | 9,8 | 10,7 % |
| 40 min. | 96,2 % | 7,0 | 7,3 % |
| 50 min. | 98,7 % | 4,1 | 4,1 % |
| 60 min. | 100,2 % | 2,2 | 2,2 % |
| 70 min. | 100,7 % | 1,8 | 1,8 % |

Protocole n° 2 :

Le produit à l'essai se présente sous forme de compresses solides obtenues suivant le procédé décrit ci-dessus. Ces compresses sont constituées de collagène (95%) et de métronidazole (5%), ces pourcentages étant exprimés en matière sèche. La libération in vitro du métronidazole est appréciée par une méthode microbiologique : recherche du pouvoir bactéricide vis-à-vis de germes anaérobies de la flore buccale connus pour leur sensibilité au métronidazole.

Pour cette détermination, quatre souches ont ainsi été testées :
— Fusobacterium nucleatum (souche 1-5)
— Veillonella alcalescens (souche BS 4)
— Bacteroïdes gingivalis (souche 1-18)
— Bacteroïdes intermedius (souche IX)

Conduite de l'essai

En milieu liquide approprié (milieu PGY enrichi de 10 pour cent de sang de cheval) on introduit un fragment de produit à tester (1 × 1cm de compresse) puis, pour chaque germe, un inoculum contenant approximativement 10⁵ germes.

Après incubation en anaérobiose discontinue, on observe une activité bactéricide de l'association vis-à-vis de l'ensemble des souches testées. L'activité bactéricide ainsi mise en évidence traduit la libération du métronidazole à partir des compresses.

## Application thérapeutique

Les préparations médicamenteuses obtenues selon le procédé de l'invention sont avant tout destinées au traitement d'affections du parodonte, et en particulier au traitement topique des poches parodontales.

Les poches parodontales sont des lésions bien localisées aux tissus entourant la racine de la dent (parodonte) ; leur traitement est essentiellement local. Le traitement étiologique comporte préférentiellement l'application in situ, c'est-à-dire dans la poche parodontale elle-même, de préparations médicamenteuses répondant à deux conditions fondamentales :

— Elles doivent contenir un ou plusieurs agents anti-infectieux actifs sur les micro-organismes reconnus comme responsables du déclenchement et de l'évolution de l'affection (germes anaérobies pathogènes et protozoaires parasites essentiellement)

— elles doivent assurer la libération in situ desdits agents anti-infectieux en concentration suffisante et sur une période suffisamment longue pour assurer la destruction des micro-organismes pathogènes.

Il est en outre souhaitable que :

1/ L'introduction de la préparation médicamenteuse dans la poche parodontale soit facile pour un praticien qualifié,

2/ le produit une fois en place, dans la poche parodontale, ne fasse pas office de corps étranger et ne provoque pas une réaction inflammatoire qui s'ajoute à celles causées par les micro-organismes responsables de la lésion initiale et par les concrétions et amas de débris divers encombrant la poche,

3/ le produit se résorbe in situ sans appeler une nouvelle intervention pour l'extraire, une fois achevée la libération du ou des principes actifs anti-infectieux.

Les associations suivant la présente invention se distinguent de tous les traitements actuellement appliqués au traitement des poches parodontales en ce qu'elles répondent à toutes les conditions énumérées ci-dessus. Elles offrent, de surcroît, des avantages appréciables liés aux propriétés biologiques du collagène natif : activité hémostatique locale et, plus encore, activité cicatrisante favorisant le comblement rapide des poches parodontales, c'est-à-dire leur évolution vers la guérison. Or, si l'ensemble des techniques classiquement appliquées au traitement des poches parodontales permet de retarder leur évolution vers l'aggravation, voire de l'arrêter (avant la chute des dents), le comblement des poches constituées ne peut être que rarement obtenu.

L'emploi systématique de préparations médicamenteuses suivant l'invention doit permettre de recourir moins souvent aux interventions chirurgicales sanglantes (gingivectomies) couramment pratiquées dans le traitement des poches parodontales. Cette application thérapeutique des préparations obtenues selon l'invention se trouve illustrée par les deux observations cliniques mentionnées ci-après.

## 1er cas clinique : Traitement d'une poche parodontale profonde

Madame Colette J..., âgée de 32 ans, par ailleurs en bonne santé, présente une parodontite à progression rapide active avec des poches profondes (de 4 mm à 9 mm), des pertes d'attache sévères (de 50% à 80%), des défauts intraosseux et des atteintes des espaces inter-radiculaires. La flore sous-gingivale est essentiellement composée de bactéries motiles (y compris de nombreux spirochètes).

Après institution d'une hygiène dentaire rigoureuse visant à l'élimination de la plaque supragingivale, des détartrages/surfaçages de la totalité du parodonte ont été effectués. Les poches très profondes ont été traitées par lambeau de pleine épaisseur afin de faciliter l'accès à la partie radiculaire de la poche. Cependant, au niveau de la face distale de la dent n° 22, pour des raisons esthétiques, il n'a pas été possible de réaliser des actes chirurgicaux. C'est la raison pour laquelle, après instrumentation "à l'aveugle" de cette poche, il a été placé une compresse réalisée selon l'exemple précité. Trois semaines après traitement, la poche résiduelle mesurait 3 mm (au lieu de 7 mm) et le gain d'attache était de 3 mm (soit 1 seul mm de récession). Il est clair, qu'un tel succès n'aurait pas pu être obtenu sans l'application in situ de la compresse selon l'invention.

2ème cas clinique : cicatrisation après chirurgie parodontale

Il a été procédé à l'élimination du tissu de granulation par "gingivectomie à biseau interne" sur le bloc incisivo-canin de Monsieur Jean M... qui présentait des poches supra-osseuses de 5 mm. L'acte a été réalisé sous anesthésie locale et a duré 25 minutes sans complication majeure (excepté un saignement quelque peu abondant). Une compresse réalisée selon l'exemple précité, déposée sur les surfaces opérées, a fait cesser le saignement et a permis de recouvrir la plaie d'un pansement chirurgical. Au jour 8, on a déposé le pansement et constaté une cicatrisation parfaite avec épithélialisation complète de la plaie (ce qui ne se produit en général qu'après 3 semaines).

## Revendications

1. Procédé de fabrication de préparations médicamenteuses solides associant du collagène et un agent anti-infectieux imidazolé, caractérisé en ce que l'on réalise les opérations successives suivantes :
   - on dissout une quantité prédéterminée d'au moins un dérivé imidazolé dans de l'eau déminéralisée,
   - on disperse directement dans la solution ainsi obtenue une quantité prédéterminée de collagène fibreux non-dénaturé, et
   - on lyophilise la dispersion ainsi obtenue.

2. Procédé selon la revendication 1, caractérisé en ce que l'on utilise un collagène insoluble dans l'eau, hydrodispersible et ne présentant qu'une faible fraction acido-soluble.

3. Procédé selon la revendication 1 ou 2, caractérisé en ce que l'on utilise, pour une partie en masse de collagène fibreux non-dénaturé 0,005 à 0,25 parties en masse de dérivé imidazolé, et 45 à 200 parties en masse d'eau déminéralisée.

4. Procédé selon l'une des revendications 1 à 3, caractérisé en ce que la dispersion de collagène dans la solution de dérivé imidazolé est effectuée dans une cuve de mélange équipée d'une turbine de défloculation.

5. Procédé selon l'une des revendications 1 à 4, caractérisé en ce que la dispersion de collagène dans la solution de dérivé imidazolé est effectuée pendant une durée supérieure ou égale à 15 minutes, à une température inférieure à 30°C.

6. Procédé selon l'une des revendications 1 à 5, caractérisé en ce que l'on répartit, à poids constant, la dispersion de collagène fibreux dans la solution de dérivé imidazolé sur des plateaux équipés de grilles de séparation, et en ce que l'on lyophilise directement lesdits plateaux ainsi préparés.

7. Procédé selon l'une des revendications 1 à 6, caractérisé en ce que la lyophilisation est réalisée dans les conditions suivantes :
   — température de congélation : $-30 \pm 5°C$,
   — durée de cycle de lyophilisation : $\geq$ à 18 heures
   — température de désorption : $28 \pm 5°C$.

8. Procédé selon l'une des revendications 1 à 7, caractérisé en ce qu'après la lyophilisation, on conditionne les préparations obtenues, puis on les stérilise par rayonnement ionisant, de préférence par irradiation gamma 25000 $m^2 \cdot s^{-2}$ (25 KGy).

9. Procédé selon l'une des revendications 1 à 8, caractérisé en ce que l'on contrôle la non-dénaturation du collagène par calorimétrie différentielle programmée et/ou par diffraction de rayons X, préalablement à la dispersion du collagène dans la solution de dérivé imidazolé, et postérieurement à l'étape de lyophilisation et/ou de stérilisation.

10. Procédé selon l'une des revendications 1 à 9, caractérisé en ce que le dérivé imidazolé utilisé est choisi dans la famille des nitro-5-imidazoles, en particulier parmi le métronidazole, l'ornidazole et le tinidazole.

11. Compositions thérapeutiques contenant une préparation obtenue par le procédé selon l'une des revendications 1 à 10.

## Patentansprüche

1. Verfahren zur Herstellung von festen Arzneimittelzusammensetzungen, die Collagen und ein anti-infektiöses Imidazol-Mittel enthalten, dadurch gekennzeichnet, daß man nacheinander folgende Arbeitsgänge durchführt :
   - man löst eine vorbestimmte Menge mindestens eines Imidazol-Derivats in entmineralisiertem Wasser,
   - man dispergiert in der so erhaltenen Lösung direkt eine vorbestimmte Menge nicht-denaturiertes faserförmiges Collagen, und

● man lyophilisiert die so erhaltene Dispersion.

2. Verfahren nach Anspruch 1, dadurch gekennzeichnet, daß man in Wasser unlösliches, hydrodispergierbares und nur eine geringe säurelösliche Fraktion aufweisendes Collagen verwendet.

3. Verfahren nach Anspruch 1 oder 2, dadurch gekennzeichnet, daß man auf einen Massenteil des nicht-denaturierten faserförmigen Collagens 0,005 bis 0,25 Massenteile Imidazol-Derivat und 45 bis 200 Massenteile entmineralisiertes Wasser verwendet.

4. Verfahren nach einem der Ansprüche 1 bis 3, dadurch gekennzeichnet, daß man das Collagen in der Lösung des Imidazol-Derivats in einem Mischgefäß dispergiert, das mit einer Entflockungs-Turbine ausgerüstet ist.

5. Verfahren nach einem der Ansprüche 1 bis 4, dadurch gekennzeichnet, daß das Collagen in der Lösung des Imidazol-Derivats während einer Dauer von über oder gleich 15 Minuten bei einer Temperatur unter 30°C dispergiert wird.

6. Verfahren nach einem der Ansprüche 1 bis 5, dadurch gekennzeichnet, daß man die Dispersion des faserförmigen Collagens in der Lösung des Imidazol-Derivats über mit Trenngittern ausgerüstete Platten verteilt, und daß man die so präparierten Platten direkt lyophilisiert.

7. Verfahren nach einem der Ansprüche 1 bis 6, dadurch gekennzeichnet, daß man unter folgenden Bedingungen lyophilisiert :
— Gefriertemperatur : –30 ± 5°C,
— Dauer des Lyophilisierungs-Zyklus : ≥ 18 Stunden,
— Desorptionstemperatur : 28 ± 5°C.

8. Verfahren nach einem der Ansprüche 1 bis 7, dadurch gekennzeichnet, daß man nach dem Lyophilisieren die erhaltenen Zusammensetzungen konditioniert und sie anschließend durch ionisierende Strahlung, vorzugsweise durch Gammastrahlung 25 000 $m^2 \cdot s^{-2}$ (25 KGy) sterilisiert.

9. Verfahren nach einem der Ansprüche 1 bis 8, dadurch gekennzeichnet, daß man die nicht-Denaturierung des Collagens durch programmierte Differenzial-Kaloriemetrie und/oder durch Röntgenstrahlen-Beugung kontrolliert, bevor das Collagen in der Lösung des Imidazol-Derivats dispergiert wird, und nach der Lyophilisierungs- und/oder Sterilisations-Stufe.

10. Verfahren nach einem der Ansprüche 1 bis 9, dadurch gekennzeichnet, daß das verwendete Imidazol-Derivat ausgewählt wird aus der Familie der 5-Nitro-imidazole, insbesondere aus dem Metronidazol, dem Ornidazol und dem Tinidazol.

11. Therapeutische Zusammensetzungen, enthaltend eine Zusammensetzung, erhalten nach dem Verfahren eines der Ansprüche 1 bis 10.

## Claims

1. A process for the production of solid medicinal preparations in which collagen and an imidazole anti-infection agent are associated, characterised in that the following successive operations are performed :
● a predetermined quantity of at least one imidazole derivative is dissolved in demineralised water,
● a predetermined quantity of non-denatured fibrous collagen is directly dispersed in the resulting solution, and
● the resulting dispersion is freeze-dried.

2. A process according to claim 1, characterised in that the collagen used is insoluble in water, hydrodispersible and has only a small acid-soluble fraction.

3. A process according to claim 1 or 2, characterised in that 0.005 to 0.25 parts by mass of imidazole derivative end 45 to 200 parts by mass of demineralised water are used per 1 part by mass of non-dernatured fibrous collagen.

4. A process according to any one of claims 1 to 3, characterised in that the dispersion of collagen in the imidazole derivative solution is carried out in a mixing tank provided with a deflocculation turbine.

5. A process according to any one of claims 1 to 4, characterised in that the dispersion of collagen in the imidazole derivative solution is carried out for a period greater than or equal to 15 minutes at a temperature below 30°C.

6. A process according to any one of claims 1 to 5, characterised in that the dispersion of fibrous collagen in the imidazole derivative solution is distributed, at a constant weight, on to plates provided with separating grids, and in that the said plates thus prepared are freeze-dried directly.

7. A process according to any one of claims 1 to 6, characterised in that freeze-drying is carried out in the following conditions :
— freezing temperature : –30 ± 5°C,

— freeze-drying cycle time : ≥ 18 hours

— desorption temperature : 28 ± 5°C.

8. A process according to any one of claims 1 to 7, chatacterised in that after freeze-drying the resulting preparations are packed and then sterilized by ionizing irradiation, preferably by 25000 $m^2 \cdot s^{-2}$ gamma irradiation (25 KGy).

9. A process according to any one of claims 1 to 8, characterised in that the non-denaturation of the collagen is checked by programmed differential calorimetry and/or by X-ray diffraction prior to the dispersion of the collagen in time imidazole derivative solution and after time freeze-drying and/or sterilisation step.

10. A process according to any one of claims 1 to 9, characterised in that the imidazole derivative used is selected from the group of nitro-5-imidazoles, more particularly metronidazole, ornidazole and linidazole.

11. Therapeutic compositions a preparation produced by the process according to any one of claims 1 to 10.